# EUROPÄISCHE PATENTSCHRIFT

(11) **EP 0 365 744 B1**
(45) Veröffentlichungstag und Bekanntmachung des Hinweises auf die Patenterteilung: **20.01.1993**
(21) Anmeldenummer: 89110432.5
(22) Anmeldetag: 09.06.1989
(51) Int. Cl.: A61K 6/00, A61K 6/08, A61K 6/04

(54) **Metall/Kunststoff-Verbundkörper und Verfahren zu seiner Herstellung**
Metal/synthetic resin compositie article, and process for its preparation
Matériau composite métal/résine synthétique et procédé pour sa préparation

(30) Priorität: 26.10.1988 DD 321100
(43) Veröffentlichungstag der Anmeldung: 02.05.1990
(73) Patentinhaber: Heraeus Kulzer GmbH, 63450 Hanau (DE)
(72) Erfinder: Göbel, Roland, Dr., DD-6900 Jena (DD); Tiller, Hans-Jürgen, Prof., DD-6900 Jena (DD); Musil, Rudolf, Prof., DD-6900 Jena (DD); Magnus, Brigitte, DD-6900 Jena (DD); Schödel, Dieter, Dr., D-6200 Wiesbaden (DE); Schmidt, Albert, D-6380 Band Homburg (DE)
(74) Vertreter: Kühn, Hans-Christian

(56) Entgegenhaltungen:
- EP-A- 0 056 269
- EP-A- 0 151 233
- DE-C- 3 642 290
- US-A- 4 364 731

## Beschreibung

### Anwendungsgebiet der Erfindung

Die Erfindung betrifft einen dentalen Metall/Kunststoff-Verbundkörper mit einer zwischen Metall und Kunststoff angeordneten, Siliciumdioxid enthaltenden Haftvermittler-Schicht und ein Verfahren und ein Mittel zu seiner Herstellung.

### Charakteristik des bekannten Standes der Technik

Bei der Herstellung von Zahnersatz, besonders von künstlichen Zahnkronen und Brücken, aus mit Kunststoff verblendeten metallischen Gerüsten aus edelmetallhaltigen oder edelmetallfreien Dentallegierungen kommt dem Verbund zwischen dem Kunststoff und dem Metall eine besondere Bedeutung zu. Zahlreiche Vorschläge sind gemacht worden, um Kunststoff und Metall haftfest miteinander zu verbinden und spaltfreie und dauerhafte Kunststoff-Verblendungen zu erzeugen.

US-A-4 364 731 betrifft die Verbesserung der Haftung zwischen dem Metallgerüst einer künstlichen Zahnkrone und der Kunststoff-Verblendung durch eine silanisierte oxidische Zwischenschicht. Die dünne Zwischenschicht kann aus Aluminiumoxid, Siliciumdioxid, einem Aluminat, Silicat, Aluminosilicat oder siliciumdioxid-reichem Glas bestehen und wird vorzugsweise durch Kathodenzerstäubung aufgebracht. Die Silanisierung der oxidischen Schicht kann zum Beispiel mit 3-Aminopropyltriethoxysilan oder Vinyltrichlorsilan erfolgen.

Aus der DE-PS 36 42 290 ist ein Verfahren zur Verbesserung der Haftung von Kunststoffen auf Metallen, insbesondere bei der Herstellung von Zahnprothesen, durch Aufbringen und anschließendes Silanisieren einer Siliciumdioxid-Schicht bekannt. Die Siliciumdioxid-Schicht wird durch Auftragen eines Kieselsols (durchschnittliche Primärteilchengröße 5 - 150 Nanometer) oder einer Dispersion einer feinstteiligen Kieselsäure (durchschnittliche Primärteilchengröße 5 - 50 Nanometer) auf die Metalloberfläche und Einbrennen bei einer Temperatur von 100 - 800 °C aufgebracht. Die Kieselsole sind wässerige kolloidale Dispersionen von amorphem Siliciumdioxid, das in Form von untereinander unvernetzten kugelförmigen, an der Oberfläche Hydroxy-Gruppen aufweisenden Einzelpartikeln vorliegt. Für die Dispersion feinstteiliger Kieselsäuren in Wasser und/oder Alkohol als Dispergiermittel sind pyrogene Kieselsäuren besonders geeignet.

Nachteilig bei den bisher bekannten Verfahren sind einerseits die ungenügende Haftfestigkeit des Kunststoffes bei der breiten Palette bekannter Dentallegierungen, insbesondere unter den Bedingungen des Mundmilieus, beziehungsweise andererseits der zu hohe apparative Aufwand zur Herstellung derartiger Verbunde.

### Ziel der Erfindung

Ziel der Erfindung ist die Entwicklung und Herstellung eines feuchte- und dauerstabilen Verbundes von Kunststoffen mit Metallen für den Einsatz in der Dentalprothetik.

### Darlegung des Wesens der Erfindung

Ausgehend von diesem Ziel, stellt sich als Aufgabe der Erfindung, mit Kunststoff verblendeten und mit einer Siliciumdioxid enthaltenden Haftvermittler-Schicht zwischen Metall und Kunststoff versehenen Zahnersatz - im folgenden als dentaler Metall/Kunststoff-Verbundkörper bezeichnet - mit verbesserter Haftfestigkeit des Kunststoffes an in der Dentaltechnik gebräuchliche Edelmetall-Legierungen und Unedelmetall-Legierungen und ein Verfahren und ein Mittel zu seiner Herstellung zu finden.

Der die Lösung der Aufgabe darstellende dentale Metall/Kunststoff-Verbundkörper ist dadurch gekennzeichnet, daß die Haftvermittler-Schicht aus Siliciumdioxid und einem oder mehreren Metalloxiden besteht und einen Konzentrationsgradienten aufweist, wobei die Metalloxid-Konzentration in der Schicht in Richtung auf den Kunststoff hin abnimmt.

Besonders bewährt hat sich der Metall/Kunststoff-Verbundkörper, wenn der Metalloxid-Gehalt der Haftvermittler-Schicht 1 - 20 % Stoffmengenanteil beträgt; bevorzugt wird ein Metalloxid-Gehalt von 2 - 5 % Stoffmengenanteil.

Die aus Siliciumdioxid und Metalloxid bestehende Haftvermittler-Schicht besitzt vorzugsweise eine Dicke von 20 - 400 Nanometer. Sie weist einen Konzentrationsgradienten auf, das heißt, in der Schicht ändert sich die Konzentration an Siliciumdioxid und Metalloxid kontinuierlich mit der Schichtdicke.

Durch die hohe Konzentration an Metalloxid in dem metallnahen Bereich der Haftvermittler-Schicht und die hohe Konzentration an Siliciumdioxid in dem kunststoffnahen Bereich der Haftvermittler-Schicht wird eine sehr gute Haftfestigkeit des Verbundes Metall/Kunststoff erzielt.

Mit der kontinuierlichen Konzentrationsänderung geht eine kontinuierliche Änderung der Struktur und anderer Eigenschaften einher, so daß durch die Haftvermittler-Schicht Spannungen zwischen Metall und Kunststoff ausgeglichen werden. Dadurch wird insbesondere für edelmetallhaltige Dentallegierungen, die an sich bei Temperaturen unter 700 °C an Silicathaftvermittlern keine beständigen Bindungen ausbilden, ein wesentlicher Fortschritt erreicht.

Besonders bewährt hat sich der Verbundkörper, wenn die Haftvermittler-Schicht in ihrem metallnahen Bereich 65 - 100 % Stoffmengenanteil, vorzugsweise 95 % Stoffmengenanteil, Metalloxid und in ihrem kunststoffnahen Bereich 0 - 10 % Stoffmengenanteil, vorzugsweise 2 % Stoffmengenanteil, Metalloxid enthält. Die Konzentrationsangaben beziehen sich auf einen 0,5 - 5 Nanometer dicken metallnahen Bereich und einen ebenfalls 0,5 - 5 Nanometer dicken kunststoffnahen Bereich der Haftvermittler-Schicht.

Als Metalloxid hat sich Chromoxid besonders bewährt.

Erfindungsgemäß ist das Verfahren zur Herstellung des dentalen Metall/Kunststoff-Verbundkörpers gekennzeichnet durch das Auftragen einer kolloidalen Dispersion von Siliciumdioxid, einer das Siliciumdioxid vernetzenden Komponente und einer oder mehrerer das Metalloxid bildender Komponenten auf die Metall-Oberfläche, das Aushärten durch Erhitzen auf 150 - 530 °C, das Auftragen eines polymerisierbaren Dentalmaterials und die Polymerisation.

Dabei kann auf die Metall-Oberfläche eine Mischung aller Komponenten aufgetragen und anschließend ausgehärtet werden. Es ist aber auch möglich, zunächst nur die Metalloxid bildende(n) Komponente(n) aufzutragen und auf 300 °C zu erhitzen (konditionieren) und dann die anderen beiden Komponenten als Mischung miteinander aufzutragen; anschließend wird durch Erhitzen auf 150 - 530 °C, vorzugsweise auf 330 - 420 °C, ausgehärtet.

Es hat sich als günstig erwiesen, die Haftvermittler-Schicht vor dem Auftragen des Dentalmaterials in an sich bekannter Weise zu silanisieren.

Das Mittel zur Herstellung des die Haftvermittler-Schicht aufweisenden dentalen Metall/Kunststoff-Verbundkörpers ist dadurch gekennzeichnet, daß es eine kolloidale Dispersion von Siliciumdioxid, eine das Siliciumdioxid vernetzende Komponente und eine oder mehrere Metalloxid bildende Komponenten umfaßt.

Der Silicium-Gehalt des Mittels beträgt 10⁻³ bis 10 Gewichts-%, der Metall-Gehalt 10⁻⁴ bis 1 Gewichts-%.

Die das Siliciumdioxid vernetzende Komponente weist einen Silicium-Gehalt von 10⁻⁵ bis 1 Gewichts-% auf. Sie besteht vorzugsweise aus einem sauren Hydrolysat eines Alkoxysilans, besonders von Tetramethoxysilan, und kann in der Dispersion enthalten sein. Das Mittel zur Herstellung der Haftvermittler-Schicht liegt dann als Gel vor, das leicht zu handhaben ist.

Die Metalloxid bildende(n) Komponente(n) liegt bzw. liegen getrennt davon, gelöst in einem Lösungsmittel, vor; bevorzugt werden Chromverbindungen, besonders Ammoniumdichromat.

Geeignete Dispergier- und Lösungsmittel sind Wasser und/oder Alkanole.

Den Dispergier- und Lösungsmitteln wird ein Benetzungsmittel, wie beispielsweise 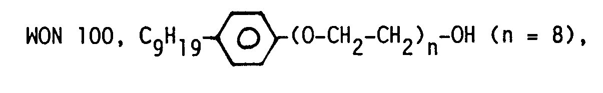
in einer Menge von 0,05 bis 0,25 % - bezogen auf das Gesamtvolumen - zugesetzt.

Die folgenden Beispiele dienen zur näheren Erläuterung der Erfindung.

### Ausführungsbeispiele

### Beispiel 1

Auf ein aus der dentalen Nickel-Chrom-Legierung NCA gefertigtes Plättchen (10 x 10 x 4 mm) wird nach Sandstrahlen und Reinigen mit Essigsäureäthylester eine erste Lösung aus

| | |
|---|---|
| 0,05 g | Ammoniumdichromat, (NH₄)₂Cr₂O₇ |
| 5 ml | Wasser |
| 5 ml | Isopropanol |

mit einem Pinsel aufgetragen. Das Plättchen wird getrocknet und 10 Minuten lang bei 400 °C konditioniert. Nach dem Abkühlen wird eine zweite Lösung aus

| | |
|---|---|
| 0,3 ml | Kieselsol (300 g SiO₂/l Wasser) |
| 0,2 ml | Tetramethoxysilan-Hydrolysat |
| 1 ml | Aceton |
| 50 ml | Isopropanol |

aufgetragen. Das Plättchen wird dann nochmals getrocknet und 3 Minuten lang bei 380 °C in einem Dentalkeramik-Ofen ausgehärtet.

Das mit der Siliciumdioxid-Chromoxid-Schicht versehene Plättchen wird dann mit einer 3-Methacryloyloxypropyltrimethoxysilan-Lösung silanisiert und nach dem Trocknen mit dem photopolymerisierbaren Kronen- und Brückenmaterial Dentacolor der Firma Kulzer GmbH verblendet. Die Photopolymerisation erfolgt in dem Dentacolor XS-Gerät der Firma Kulzer GmbH.

### Beispiel 2

Auf ein aus der dentalen Silber-Palladium-Legierung Palliag gefertigtes Plättchen (10 x 10 x 2 mm) wird nach Sandstrahlen und Reinigen mit Essigsäureäthylester eine Mischung aus 1 Gewichts-Teil der Lösung aus

| | |
|---|---|
| 0,05 g | Ammoniumdichromat, (NH₄)₂Cr₂O₇ |
| 5 ml | Wasser |
| 5 ml | Isopropanol |

und 20 Gewichts-Teilen der Lösung aus

| | |
|---|---|
| 0,3 ml | Kieselsol (300 g SiO₂/l Wasser) |
| 0,2 ml | Tetramethoxysilan-Hydrolysat |
| 1 ml | Aceton |
| 50 ml | Isopropanol |

mit dem Pinsel aufgetragen. Das Plättchen wird getrocknet und 4 Minuten lang bei 380 °C ausgehärtet. Das mit der Siliciumdioxid-Chromoxid-Schicht versehene Plättchen wird dann mit einer 3-Methacryloyloxypropyltrimethoxysilan-Lösung silanisiert und nach dem Trocknen mit dem photopolymerisierbaren Kronen- und Brückenmaterial Dentacolor der Firma Kulzer GmbH verblendet. Die Photopolymerisation erfolgt in dem Dentacolor XS-Gerät der Firma Kulzer GmbH.

### Beispiel 3

Auf ein aus der dentalen Gold-Legierung Degulor M gefertigtes Plättchen (10 x 10 x 1 mm) wird nach Sandstrahlen und Reinigen mit Essigsäureäthylester eine Mischung aus 1 Gewichts-Teil der Lösung aus

| | |
|---|---|
| 0,05 g | Ammoniumdichromat, (NH₄)₂Cr₂O₇ |
| 5 ml | Wasser |
| 5 ml | Isopropanol |

und 20 Gewichts-Teilen der Lösung aus

| | |
|---|---|
| 0,3 ml | Kieselsol (300 g SiO₂/l Wasser) |
| 0,2 ml | Tetramethoxysilan-Hydrolysat |
| 1 ml | Aceton |
| 50 ml | Isopropanol |

mit dem Pinsel aufgetragen. Das Plättchen wird getrocknet und 4 Minuten lang bei 380 °C ausgehärtet. Das mit der Siliciumdioxid-Chromoxid-Schicht versehene Plättchen wird dann mit einer 3-Methacryloyloxypropyltrimethoxysilan-Lösung silanisiert und nach dem Trocknen mit dem photopolymerisierbaren Kronen- und Brückenmaterial Dentacolor der Firma Kulzer GmbH verblendet. Die Photopolymerisation erfolgt in dem Dentacolor XS-Gerät der Firma Kulzer GmbH.

### Haftfestigkeit

Die Prüfung der Haftfestigkeit der so hergestellten Verbundkörper aus Dentallegierung und Kunststoff-Verblendung erfolgt durch Bestimmung der Scherfestigkeit nach einem Feuchtebelastungstest unter extremen Bedingungen. Dazu werden die Verbundkörper 30 Minuten lang in kochendem Wasser gelagert und anschließend in einer Abschervorrichtung (0,5 cm/min Stempelvorschub) geprüft.

Die an den Verbundkörpern gemäß der Erfindung und - zum Vergleich dazu - an Verbundkörpern aus den genannten Dentallegierungen und dem Kronen- und Brückenmaterial Dentacolor ohne Haftvermittler-Schicht und mit der Haftvermittler-Schicht aus Siliciumdioxid (DE-PS 36 42 290) gemessenen Scherfestigkeitswerte werden in der Tabelle angegeben.

**Tabelle**

| Legierung | Scherfestigkeit [MPa] | | |
|---|---|---|---|
| | ohne Haftvermittler-Schicht | Siliciumioxid-Haftvermittler-Schicht | Siliciumioxid/Chromoxid-Haftvermittler-Schicht |
| NCA (Ni-Cr) | < 5 | 12 | 16 |
| Palliag (Ag - Pd) | < 8 | | 26 |
| Degulor (Au) | < 6 | 8 | 25 |

## Patentansprüche

1. Dentaler Metall/Kunststoff-Verbundkörper mit einer zwischen Metall und Kunststoff angeordneten, Siliciumdioxid enthaltenden Haftvermittler-Schicht, dadurch gekennzeichnet, daß die Haftvermittler-Schicht aus Siliciumdioxid und einem oder mehreren Metalloxiden besteht und einen Konzentrationsgradienten aufweist, wobei die Metalloxid-Konzentration in der Schicht in Richtung auf den Kunststoff hin abnimmt.

2. Verbundkörper nach Anspruch 1, dadurch gekennzeichnet, daß der Metalloxid-Gehalt der Haftvermittler-Schicht 1 - 20 % Stoffmengenanteil beträgt.

3. Verbundkörper nach Anspruch 2, dadurch gekennzeichnet, daß der Metalloxid-Gehalt 2 - 5 % Stoffmengenanteil beträgt.

4. Verbundkörper nach einem der Ansprüche 1 bis 3, dadurch gekennzeichnet, daß der 0,5 - 5 Nanometer dicke metallnahe Bereich der Haftvermittler-Schicht 65 - 100 % Stoffmengenanteil Metalloxid enthält.

5. Verbundkörper nach Anspruch 4, dadurch gekennzeichnet, daß der 0,5 - 5 Nanometer dicke metallnahe Bereich 95 % Stoffmengenanteil Metalloxid enthält.

6. Verbundkörper nach einem der Ansprüche 1 bis 5, dadurch gekennzeichnet, daß der 0,5 - 5 Nanometer dicke kunststoffnahe Bereich der Haftvermittler-Schicht 0 - 10 % Stoffmengenanteil Metalloxid enthält.

7. Verbundkörper nach Anspruch 6, dadurch gekennzeichnet, daß der 0,5 - 5 Nanometer kunststoffnahe Bereich 2 % Stoffmengenanteil Metalloxid enthält.

8. Verbundkörper nach einem der Ansprüche 1 bis 7, dadurch gekennzeichnet, daß das Metalloxid Chromoxid ist.

9. Verfahren zur Herstellung des dentalen Metall/Kunststoff-Verbundkörpers mit einer zwischen Metall und Kunststoff angeordneten, Siliciumdioxid enthaltenden Haftvermittler-Schicht nach den Ansprüchen 1 bis 8 durch Auftragen einer kolloidalen Dispersion von Siliciumdioxid auf die Metalloberfläche, Erhitzen auf eine Temperatur von 100 - 800 °C, Auftragen von polymerisierbarem Dentalmaterial und Polymerisation, dadurch gekennzeichnet, daß zur Erzeugung einer einen Konzentrationsgradienten aufweisenden Haftvermittler-Schicht die kolloidale Dispersion von Siliciumdioxid, eine das Siliciumdioxid vernetzende Komponente und eine oder mehrere Metalloxid bildende Komponenten auf die Metalloberfläche aufgetragen und bei einer Temperatur von 150 - 530 °C ausgehärtet werden.

10. Verfahren nach Anspruch 9, dadurch gekennzeichnet, daß die kolloidale Dispersion von Siliciumdioxid und die das Siliciumdioxid vernetzende Komponente zusammen einen Silicium-Gehalt von 10⁻³ bis 10 Gewichts-% aufweisen und der Metall-Gehalt der Metalloxid bildende(n) Komponente(n) 10⁻⁴ bis 1 Gewichts-% beträgt.

11. Verfahren nach Anspruch 10, dadurch gekennzeichnet, daß die das Siliciumdioxid vernetzende Komponente einen Silicium-Gehalt von 10⁻⁵ bis 1 Gewichts-% aufweist.

12. Verfahren nach einem der Ansprüche 9 bis 11, dadurch gekennzeichnet, daß als Siliciumdioxid vernetzende Komponente ein Hydrolysat eines Alkoxysilans verwendet wird.

13. Verfahren nach Anspruch 12, dadurch gekennzeichnet, daß als Alkoxysilan Tetramethoxysilan verwendet wird.

14. Verfahren nach einem der Ansprüche 9 bis 13, dadurch gekennzeichnet, daß die das Siliciumdioxid vernetzende Komponente der Dispersion zugesetzt wird.

15. Verfahren nach einem der Ansprüche 9 bis 14, dadurch gekennzeichnet, daß als Metalloxid bildende Komponente eine Chromverbindung verwendet wird.

16. Verfahren nach Anspruch 15, dadurch gekennzeichnet, daß die Chromverbindung in einem Lösungsmittel gelöst wird.

17. Verfahren nach Anspruch 15 oder 16, dadurch gekennzeichnet, daß als Chromverbindung Ammoniumdichromat verwendet wird.

18. Verfahren nach einem der Ansprüche 9 bis 17, dadurch gekennzeichnet, daß als Dispergiermittel und Lösungsmittel Wasser und/oder Alkanole eingesetzt werden.

19. Verfahren nach Anspruch 18, dadurch gekennzeichnet, daß dem Dispergiermittel und Lösungsmittel ein Benetzungsmittel, wie beispielsweise 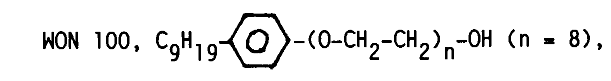 in einer Menge von 0,05 bis 0,25 % - bezogen auf das Gesamtvolumen - zugesetzt wird.

20. Verfahren nach einem der Ansprüche 9 bis 19, dadurch gekennzeichnet, daß die Haftvermittler-Schicht vor dem Auftragen des Dentalmaterials silanisiert wird.

21. Verfahren nach einem der Ansprüche 9 bis 20, dadurch gekennzeichnet, daß das Aushärten bei einer Temperatur von 330 bis 420 °C erfolgt.

22. Eine kolloidale Dispersion von Siliciumdioxid umfassendes Mittel zur Herstellung eines dentalen Metall/Kunststoff-Verbundkörpers mit einer zwischen Metall und Kunststoff angeordneten, Siliciumdioxid enthaltenden Haftvermittler-Schicht, dadurch gekennzeichnet, daß das der Erzeugung einer einen Konzentrationsgradienten aufweisenden Haftvermittler-Schicht dienende Mittel aus der kolloidalen Dispersion von Siliciumdioxid, einer das Siliciumdioxid vernetzenden Komponente und einer oder mehreren Metalloxid bildenden Komponenten besteht.

23. Mittel nach Anspruch 22, gekennzeichnet durch einen Silicium-Gehalt von 10⁻³ bis 10 Gewichts-% und einen Metall-Gehalt von 10⁻⁴ bis 1 Gewichts-%.

24. Mittel nach Anspruch 23, dadurch gekennzeichnet, daß die das Siliciumdioxid vernetzende Komponente einen Silicium-Gehalt von 10⁻⁵ bis 1 Gewichts-% aufweist.

25. Mittel nach einem der Ansprüche 22 bis 24, dadurch gekennzeichnet, daß die das Siliciumdioxid vernetzende Komponente ein Hydrolysat eines Alkoxysilans ist.

26. Mittel nach Anspruch 25, dadurch gekennzeichnet, daß das Alkoxysilan Tetramethoxysilan ist.

27. Mittel nach einem der Ansprüche 22 bis 26, dadurch gekennzeichnet, daß die das Siliciumdioxid vernetzende Komponente in der Dispersion enthalten ist.

28. Mittel nach einem der Ansprüche 22 bis 27, dadurch gekennzeichnet, daß die Metalloxid bildende Komponente eine Chromverbindung ist.

29. Mittel nach Anspruch 28, dadurch gekennzeichnet, daß die Chromverbindung in einem Lösungsmittel gelöst ist.

30. Mittel nach Anspruch 28 oder 29, dadurch gekennzeichnet, daß die Chromverbindung Ammoniumdichromat ist.

31. Mittel nach einem der Ansprüche 22 bis 30, dadurch gekennzeichnet, daß Dispergiermittel und Lösungsmittel aus Wasser und/oder Alkanolen bestehen.

32. Mittel nach einem der Ansprüche 22 bis 31, dadurch gekennzeichnet, daß es ein Benetzungsmittel, wie beispielsweise 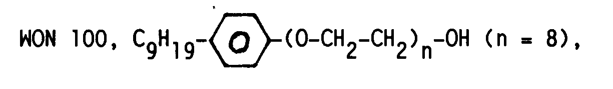 in einer Menge von 0,05 bis 0,25 % - bezogen auf das Gesamtvolumen - enthält.

## Claims

1. A dental metal/plastics composite body with an adhesive agent layer, containing silicon dioxide, arranged between metal and plastics, characterised in that the adhesive agent layer consists of silicon dioxide and one or more metal oxides and has a concentration gradient in which the metal oxide concentration in the layer decreases towards the plastics.

2. A composite body according to Claim 1, characterised in that the metal oxide content of the adhesive agent layer is 1 - 20 % substance constituent amount.

3. A composite body according to Claim 2, characterised in that the metal oxide content is 2 - 5 % substance constituent amount.

4. A composite body according to one of Claims 1 to 3, characterised in that the 0.5 - 5 nanometres thick region of the adhesive agent layer close to the metal contains 65 - 100 %, as a proportion of the material, of metal oxide.

5. A composite body according to Claim 4, characterised in that the 0.5 - 5 nanometres thick region close to the metal contains 95 %, as a proportion of the material, of metal oxide.

6. A composite body according to one of Claims 1 to 5, characterised in that the 0.5 - 5 nanometres thick region of the adhesive agent layer close to the plastics, contains 0 - 10 %, as a proportion of the material, of metal oxide.

7. A composite body according to Claim 6, characterised in that the 0.5 - 5 nanometres thick region close to the plastics contains 2 %, as a proportion of the material, of metal oxide.

8. A composite body according to one of Claims 1 to 7, characterised in that the metal oxide is chromium oxide.

9. A method for the production of the dental metal/plastics composite body with an adhesive agent layer, containing silicon dioxide, arranged between metal and plastics, according to Claims 1 to 8, through the application of a colloidal dispersion of silicon dioxide onto the metal surface, heating to a temperature of 100 - 800° C, application of polymerisable dental material and polymerisation, characterised in that for the production of an adhesive agent layer having a concentration gradient, the colloidal dispersion of silicon dioxide, a component cross-linking the silicon dioxide, and one or more components forming metal oxide, are applied onto the metal surface and are hardened at a temperature of 150 - 530° C.

10. A method according to Claim 9, characterised in that the colloidal dispersion of silicon dioxide and the component cross-linking the silicon dioxide, together have a silicon content of 10⁻³ to 10 percentage by weight, and the metal content of the component(s) forming metal oxide is 10⁻⁴ to 1 percentage by weight.

11. A method according to Claim 10, characterised in that the component cross-linking the silicon dioxide has a silicon content of 10⁻⁵ to 1 percentage by weight.

12. A method according to one of Claims 9 to 11, characterised in that a hydrolysate of an alkoxysilane is used as the component cross-linking silicon dioxide.

13. A method according to Claim 12, characterised in that tetramethoxysilane is used as alkoxysilane.

14. A method according to one of Claims 9 to 13, characterised in that the component cross-linking the silicon dioxide is added to the dispersion.

15. A method according to one of Claims 9 to 14, characterised in that a chromium compound is used as component forming metal oxide.

16. A method according to Claim 15, characterised in that the chromium compound is dissolved in a solvent.

17. A method according to Claim 15 or 16, characterised in that ammonium dichromate is used as chromium compound.

18. A method according to one of Claims 9 to 17, characterised in that water and/or alkanols are used as dispersing agent and solvent.

19. A method according to Claim 18, characterised in that a wetting agent, such as, for example 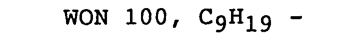 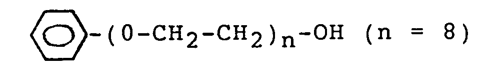 is added to the dispersing agent and solvent in a quantity of 0.05 to 0.25 % based on the total volume.

20. A method according to one of Claims 9 to 19, characterised in that the adhesive agent layer is silanised before the application of the dental material.

21. A method according to one of Claims 9 to 20, characterised in that the hardening takes place at a temperature of 330 to 420° C.

22. A colloidal dispersion of agent comprising silicon dioxide for the production of a dental metal/plastics composite body with an adhesive agent layer, containing silicon dioxide, arranged between metal and plastics, characterised in that the agent serving for the production of an adhesive agent layer having a concentration gradient consists of the colloidal dispersion of silicon dioxide, a component cross-linking the silicon dioxide and one or more components forming metal oxide.

23. An agent according to Claim 22, characterised by a silicon content of 10⁻³ to 10 percentage by weight and a metal content of 10⁻⁴ to 1 percentage by weight.

24. An agent according to Claim 23, characterised in that the component cross-linking the silicon dioxide has a silicon content of 10⁻⁵ to 1 percentage by weight.

25. An agent according to one of Claims 22 to 24, characterised in that the component cross-linking silicon dioxide is a hydrolysate of an alkoxysilane.

26. An agent according to Claim 25, characterised in that the alkoxysilane is tetramethoxysilane.

27. An agent according to one of Claims 22 to 26, characterised in that the component cross-linking silicon dioxide is contained in the dispersion.

28. An agent according to one of Claims 22 to 27, characterised in that the component forming metal oxide is a chromium compound.

29. An agent according to Claim 28, characterised in that the chromium compound is dissolved in a solvent.

30. An agent according to Claim 28 or 29, characterised in that the chromium compound is ammonium dichromate.

31. An agent according to one of Claims 22 to 30, characterised in that the dispersing agent and solvent consist of water and/or alkanols.

32. An agent according to one of Claims 22 to 31, characterised in that it contains a wetting agent, such as, for example, 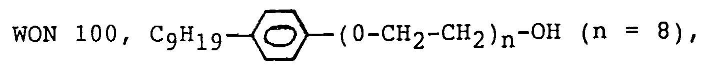 in a quantity of 0.05 to 0.25 % in relation to the total volume.

## Revendications

1. Corps composite dentaire métal/matière plastique ayant une couche d'adhésif contenant du dioxyde de silicium disposée entre le métal et la matière plastique, caractérisé en ce que la couche d'adhésif consiste en dioxyde de silicium et 1 ou plusieurs oxydes métalliques et présente un gradient de concentration, la concentration en oxyde métallique dans la couche diminuant en direction de la matière plastique.

2. Corps composite selon la revendication 1, caractérisé en ce que la teneur en oxyde métallique de la couche d'adhésif est de 1-20 %.

3. Corps composite selon la revendication 2, caractérisé en ce que la teneur en oxyde métallique est de 2-5 %.

4. Corps composite selon l'une des revendications 1 à 3, caractérisé en ce que le domaine de la couche d'adhésif voisin du métal de 0,5-5 nm d'épaisseur contient 65-100 % d'oxyde métallique.

5. Corps composite selon la revendication 4, caractérisé en ce que le domaine de 0,5-5 nm d'épaisseur voisin du métal contient 95 % d'oxyde métallique.

6. Corps composite selon l'une des revendications 1 à 5, caractérisé en ce que le domaine de 0,5-5 nm de la couche d'adhésif voisin de la matière plastique contient 0-10 % d'oxyde métallique.

7. Corps composite selon la revendication 6, caractérisé en ce que le domaine de 0,5-5 nm voisin de la matière plastique contient 2 % d'oxyde métallique.

8. Corps composite selon l'une des revendications 1 à 7, caractérisé en ce que l'oxyde métallique est l'oxyde de chrome.

9. Procédé pour la fabrication du corps composite dentaire métal/matière plastique ayant une couche d'adhésif contenant du dioxyde de silicium disposée entre le métal et la matière plastique selon l'une des revendications 1 à 8, par application d'une dispersion colloïdale de dioxyde de silicium sur la surface du métal, chauffage à une température de 100-800°C, application de la matière dentaire polymérisable et polymérisation, caractérisé en ce que, pour la fabrication d'une couche d'adhésif présentant un gradient de concentration, on applique sur la surface du métal la dispersion colloïdale de dioxyde de silicium, un composant réticulant le dioxyde de silicium et un ou plusieurs composants formant l'oxyde métallique et on durcit à une température de 150-530°C.

10. Procédé selon la revendication 9, caractérisé en ce que la dispersion colloïdale de dioxyde de silicium et le composant réticulant le dioxyde de silicium présentent ensemble une teneur en silicium de 10⁻³ à 10 % en poids et la teneur en métal du ou des composants formant l'oxyde métallique est de 10⁻⁴ à 1 % en poids.

11. Procédé selon la revendication 10, caractérisé en ce que le composant réticulant le dioxyde de silicium présente une teneuren silicium de 10⁻⁵ à 1 % en poids.

12. Procédé selon l'une des revendications 9 à 11, caractérisé en ce que l'on utilise comme composant réticulant le dioxyde de silicium un hydrolysat d'un alcoxysilane.

13. Procédé selon la revendication 12, caractérisé en ce que l'on utilise comme alcoxysilane le tétraméthoxysilane.

14. Procédé selon l'une des revendications 9 à 13, caractérisé en ce que le composant réticulant le dioxyde de silicium est ajouté à la dispersion.

15. Procédé selon l'une des revendications 9 à 14, caractérisé en ce que l'on utilise comme composant formant l'oxyde métallique un composé de chrome.

16. Procédé selon la revendication 15, caractérisé en ce que le composé de chrome est dissous dans un solvant.

17. Procédé selon la revendication 15 ou 16, caractérisé en ce que l'on utilise comme composé de chrome le bichromate d'ammonium.

18. Procédé selon l'une des revendications 9 à 17, caractérisé en ce que l'on utilise comme agent dispersant et solvant l'eau et/ou les alcanols.

19. Procédé selon la revendication 18, caractérisé en ce que l'on ajoute à l'agent dispersant et solvant un agent mouillant tel que, par exemple, 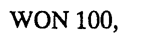 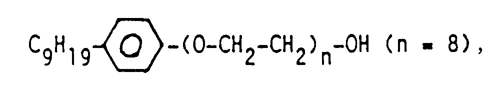 en quantité de 0,05 à 0,25 % par rapport au volume total.

20. Procédé selon l'une des revendications 9 à 19, caractérisé en ce que la couche d'adhésif est silanisée avant application de la matière dentaire.

21. Procédé selon l'une des revendications 9 à 20, caractérisé en ce que le durcissement s'effectue à une température de 330 à 420°C.

22. Produit comprenant une dispersion colloïdale de dioxyde de silicium pour la fabrication d'un corps composite dentaire métal/matière plastique ayant une couche d'adhésif contenant du dioxyde de silicium disposée entre le métal et la matière plastique, caractérisée en ce que le produit servant à la production d'une couche d'adhésif présentant un gradient de concentration consiste en la dispersion colloïdale de dioxyde de silicium, un composant réticulant le dioxyde de silicium et un ou plusieurs composants formant l'oxyde métallique.

23. Produit selon la revendication 22, caractérisé par une teneur en silicium de 10⁻³ à 10 % en poids et une teneur en métal de 10⁻⁴ à 1 % en poids.

24. Produit selon la revendication 23, caractérisé en que le composant réticulant le dioxyde de silicium présente une teneur en silicium de 10⁻⁵ à 1 % en poids.

25. Procédé selon l'une des revendications 22 à 24, caractérisé en ce que le composant réticulant le dioxyde de silicium est un hydrolysat d'un alcoxysilane.

26. Produit selon la revendication 25, caractérisé en ce que l'alcoxysilane est le tétraméthoxysilane.

27. Produit selon l'une des revendications 22 à 26, caractérisé en ce que le composant réticulant le dioxyde de silicium est contenu dans la dispersion.

28. Produit selon l'une des revendications 22 à 27, caractérisé en ce que le composant formant l'oxyde métallique est un composé de chrome.

29. Produit selon la revendication 28, caractérisé en ce que le composé de chrome est dissous dans un solvant.

30. Produit selon la revendication 28 ou 29, caractérisé en ce que le composé de chrome est le bichromate d'ammonium.

31. Produit selon l'une des revendications 22 à 30, caractérisé en ce que l'agent dispersant et solvant consiste en eau et/ou alcanol.

32. Produit selon l'une des revendications 22 à 31, caractérisé en ce qu'il contient un agent mouillant tel que, par exemple, 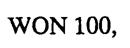 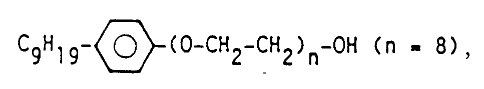 en quantité de 0,05 à 0,25 % par rapport au volume total.
